(19) Europäisches Patentamt
European Patent Office
Office européen des brevets

(11) Veröffentlichungsnummer: **0 206 159 B1**

(12) # EUROPÄISCHE PATENTSCHRIFT

(45) Veröffentlichungstag der Patentschrift: **20.02.91**

(21) Anmeldenummer: **86108069.5**

(22) Anmeldetag: **12.06.86**

Die Akte enthält technische Angaben, die nach dem Eingang der Anmeldung eingereicht wurden und die nicht in dieser Patentschrift enthalten sind.

(51) Int. Cl.5: **C08G 18/81**, C08G 18/48, G03C 1/72, G03F 7/004, H01B 3/42, H01B 3/36, C08F 283/00, G02F 1/133, G02F 1/11

(54) **Verfahren zur Herstellung wärmebeständiger strukturierter Schichten und deren Verwendung.**

(30) Priorität: **24.06.85 DE 3522507**

(43) Veröffentlichungstag der Anmeldung: **30.12.86 Patentblatt 86/52**

(45) Bekanntmachung des Hinweises auf die Patenterteilung: **20.02.91 Patentblatt 91/08**

(84) Benannte Vertragsstaaten: **AT BE CH DE FR GB IT LI LU NL SE**

(56) Entgegenhaltungen:
**EP-A- 0 021 824**
**US-A- 3 776 889**
**US-A- 4 287 323**
**US-A- 4 320 221**

(73) Patentinhaber: **Siemens Aktiengesellschaft**
**Wittelsbacherplatz 2**
**D-8000 München 2(DE)**

(72) Erfinder: **Ahne, Hellmut, Dr. Dipl.-Chem.**
**Heidestrasse 6**
**D-8551 Röttenbach(DE)**
Erfinder: **Plundrich, Winfried**
**Dürerstrasse 14**
**D-8501 Kalchreuth(DE)**

EP 0 206 159 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung wärmebeständiger strukturierter Schichten durch Auftragen strahlungsempfindlicher löslicher Polymerer in Form einer Schicht oder Folie auf ein Substrat, Bestrahlen der Schicht bzw. Folie durch Negativvorlagen mit aktinischem Licht oder durch Führen eines Licht-, Elektronen-, Laser- oder Ionenstrahls, Entfernen der nicht-bestrahlten Schicht- bzw. Folienteile und gegebenenfalls durch nachfolgendes Tempern, unter Verwendung von Photopolymeren auf Polyetherbasis in Form von Additionsprodukten aus einem olefinisch ungesättigten Monoisocyanat und einem hydroxylgruppenhaltigen Phenoxyharz, sowie die Verwendung dieser strukturierten Schichten.

Verfahren zur Herstellung strukturierter Schichten auf der Basis wärmebeständiger Polymerer sind beispielsweise aus der DE-PS 2 308 830 sowie aus der EP-PS 0 019 123 und der EP-PS 0 026 820 bekannt. Bei diesen Verfahren werden lösliche photoreaktive Vorstufen hochwärmebeständiger Polymerer zur photolithographischen Strukturierung eingesetzt und die daraus hergestellten Strukturen in einem anschließenden Temperschritt zu hochwärmebeständigen Strukturen cyclisiert. Für die vollständige Cyclisierung und die Entfernung der Spaltprodukte werden Temperaturen bis zu 400 °C benötigt. Dies erfordert thermisch hochbelastbare Substrate.

Aus der US-PS 3 776 889 sind lichtempfindliche, in organischen Lösungsmitteln lösliche, filmbildende Polymere bekannt, die im wesentlichen aus einem Allylcarbamylester eines hydroxylgruppenhaltigen Polymeren bestehen, das ein Molekulargewicht von 500 bis 115000 aufweist. Als hydroxylgruppenhaltige Polymere dienen dabei Phenoxyharze, Polyvinylalkohol, Polyester, Polyurethanharze und Schellack. Diese hydroxylgruppenhaltigen Polymeren werden mit Allylisocyanat verestert, wobei die lichtempfindlichen Polymeren entstehen. Die lichtempfindlichen Polymeren gelangen in photomechanischen Prozessen und in der Photolithographie zum Einsatz, d.h. zur Herstellung photographischer Resistbilder.

Auf dem Schaltungs- und Leitungssektor, beispielsweise in der Leiterplattentechnik, werden unter anderem Substrate auf Epoxidbasis verwendet, die thermisch maximal bis zu ca. 150 °C/1 h belastet werden können und die nur im Sekundenbereich Temperaturen von ca. 260 °C standhalten müssen, beispielsweise bei Lötprozessen. Die hier zur partiellen Leitbahnabdeckung eingesetzten sogenannten Lötstopplacke müssen ähnlichen thermischen Anforderungen entsprechen, d.h. hierbei werden zur Abdeckung der Stellen der Schaltungsoberfläche, die nicht mit dem Lotmetall in Kontakt treten sollen, Polymere mit einer mittleren thermischen Beständigkeit benötigt. Die bisher zu diesem Zweck (noch) eingesetzten Trockenresists bzw. Siebdrucklacke auf Epoxid- und Acrylatbasis entsprechen zwar den Anforderungen einer Lötstoppmaske, erfüllen jedoch nur zum Teil die gestiegenen Anforderungen an die Maßgenauigkeit in der Feinstleitertechnik mit Strukturen < 100 µm, sowie die geforderte Cycelfestigkeit. Hierfür werden photolithographische Lacksysteme benötigt.

Es ist zwar bereits ein photostrukturierbares Lacksystem auf Epoxidbasis mit in die Polymerkette eingebauten Chalkongruppen verfügbar, das eine ausreichende Maßgenauigkeit gewährleistet. Was die Photostrukturierbarkeit anbetrifft sind hierbei jedoch relativ lange Belichtungszeiten und insbesondere lange Entwicklungszeiten erforderlich. Darüber hinaus kann mit dem bekannten Lacksystem ein zusätzlicher dauerhafter Schutz der gegen Schadgas empfindlichen Schaltungsoberfläche, wie er oft gefordert wird, nur durch eine aufwendige Mehrfachbeschichtung erreicht werden. Ferner ist der Prozeßablauf, bedingt durch mehrstündige Nachhärtungszeiten, lang und kostenintensiv.

Aufgabe der Erfindung ist es, ein Verfahren der eingangs genannten Art, bei dem Photopolymere auf Polyetherbasis zum Einsatz gelangen, in der Weise auszugestalten, daß es möglich ist, dimensionsgenaue, qualitativ hochwertige strukturierte Schichten, insbesondere auf Schaltungsoberflächen, in einem Beschichtungsvorgang herzustellen, die auch den umfangreichen thermischen und mechanischen Beanspruchungen, beispielsweise bei Tauchlötprozessen, standhalten und darüber hinaus die Schaltungsoberfläche wirksam und dauerhaft gegen Feuchte und Korrosion schützen. Insbesondere soll der Prozeßdurchlauf durch kurze Belichtungs-, Entwicklungs- und Temperzeiten verkürzt und dadurch kostengünstig gestaltet werden.

Dies wird erfindungsgemäß dadurch erreicht, daß bei den Photopolymeren auf Polyetherbasis, welche in Form von Additionsprodukten aus einem olefinisch ungesättigten Monoisocyanat und einem hydroxylgruppenhaltigen Phenoxyharz vorliegen, als olefinisch ungesättigtes Monoisocyanat ein methacrylatgruppenhaltiges Isocyanat oder ein Additionsprodukt von Hydroxyethyl(meth)-acrylat an 2.4-Diisocyanatotoluol eingesetzt wird.

Das erfindungsgemäße Verfahren erlaubt - im Rahmen eines kostengünstigen Prozeßablaufes - die Herstellung feinstrukturierter Schutz- und Isolierschichten für den Halbleiter- und Schaltungssektor, die die gegen Korrosion empfindlichen Bauteile und Schaltungen dauerhaft wirksam schützen. Besonders vorteilhaft ist dabei, daß der Entwicklungsprozeß nicht zum Unterlösen führt und somit bei Lötprozessen keine Lotbrücken auftreten. Das erfindungsgemäße Verfahren erfüllt ferner nicht nur die hinsichtlich der Maßgenauigkeit der erzeugten Strukturen ge stellten Anforderungen, sondern es ermöglicht darüber hinaus eine sehr hohe Auflösung in einem breiten Schichtdickenbereich mit kurzen Entwicklungszeiten. Dieses Verfahren ist ferner kostengünstig, insbesondere auch deshalb, weil es bei einmaligem Auftrag (mit konventionellem Equipment) - gegebenenfalls nach kurzzeitiger Temperung - photostrukturierte Schichten mit ausreichend hoher Wärmebeständigkeit ergibt, die - auch unter Lötbadbedingungen - formstabil und rißfrei bleiben und als dauerhafter Schutz gegen Feuchte und Korrosion wirksam sind. Die guten elektrischen Kennwerte werden dabei auch im Feuchteklima nicht beeinträchtigt.

Beim erfindungsgemäßen Verfahren können die Photopolymeren vorteilhaft zusammen mit licht- oder strahlungsempfindlichen copolymerisationsfähigen Verbindungen eingesetzt werden. Dazu werden vorzugsweise acrylat- und methacrylatgruppenhaltige Verbindungen verwendet, insbesondere Trimethylolpropantriacrylat und -trimethacrylat und/oder 1.4-Butandioldimethacrylat. Es können aber auch allylgruppenhaltige Verbindungen, beispielsweise Diallyl-und Triallylcyanurate, sowie N-substituierte Maleinimide eingesetzt werden. Weiter können auch Photoinitiatoren und/oder -sensibilisatoren verwendet werden (vgl.: "Industrie Chimique Belge", Vol. 24, 1959, Seiten 739 bis 764, sowie J.Kosar, "Light-Sensitive Systems", John Wiley & Sons Inc., New York 1965, Seiten 143 bis 146 und 160 bis 188). Besonders geeignet sind $\alpha$-Halogenacetophenone, Dialkoxyacetophenone, wie Dimethoxy-und Diethoxyacetophenon, Benzoylphosphinoxide, die gegebenenfalls substituiert sein können, und Michler's Keton. Als Photoinitiatoren bzw. -sensibilisatoren eignen sich beispielsweise aber auch Benzoinether, 4.4'-Bis(diethylamino)-ben-zophenon, 2.6-Bis(p-azidobenzyliden)-4-methylcyclohexanon, Thioxanthone, wie Isopropylthioxanthon, und Acetophenon. Ferner können beim erfindungsgemaßen Verfahren vorteilhaft Haftvermittler verwendet werden. Dazu dienen insbesondere Silane, wie Vinyltriethoxysilan, Vinyl-tris($\beta$-methoxyethoxy)-silan, $\gamma$-Meth acryloxypropyl-trimethoxysilan, $\gamma$-Glycidoxypropyl-trimethoxy-silan und $\gamma$-Aminopropyl-triethoxysilan.

Die beim erfindungsgemäßen Verfahren verwendeten Photopolymeren sind in der EP-0S 0 206 158 beschrieben. Bevorzugt eingesetzte Photopolymere sind dabei Additionsprodukte von Isocyanatoethylmethacrylat und Phenoxyharzen, welche gegebenenfalls fluorierte Isopropylgruppen aufweisen, oder Additionsprodukte von Phenoxyharzen mit olefinisch ungesättigten Monoisocyanaten in Form von Additionsprodukten aus 2.4-Diisocyanatotoluol und Hydroxyethylacrylat oder -methacrylat. Derartige Photopolymere sind in den nachfolgenden Formeln beispielhaft verdeutlicht:

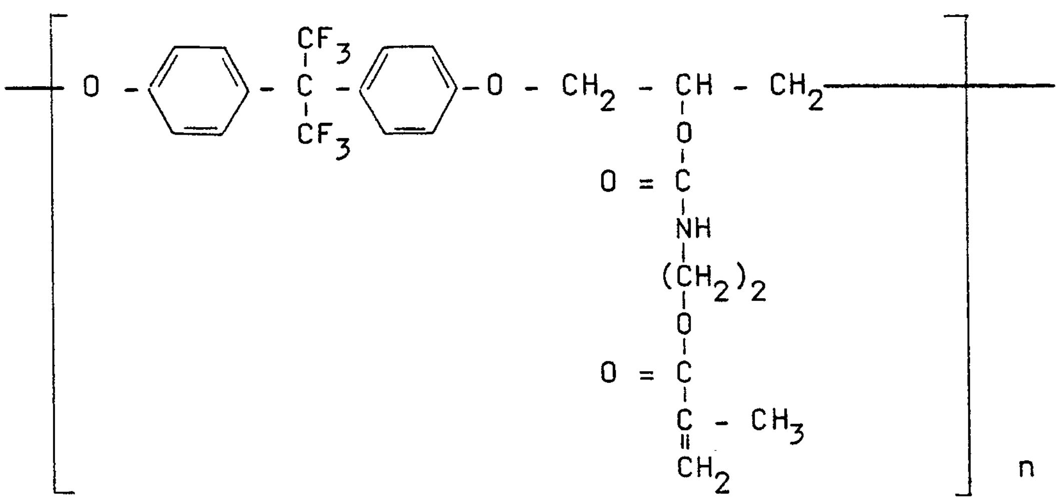

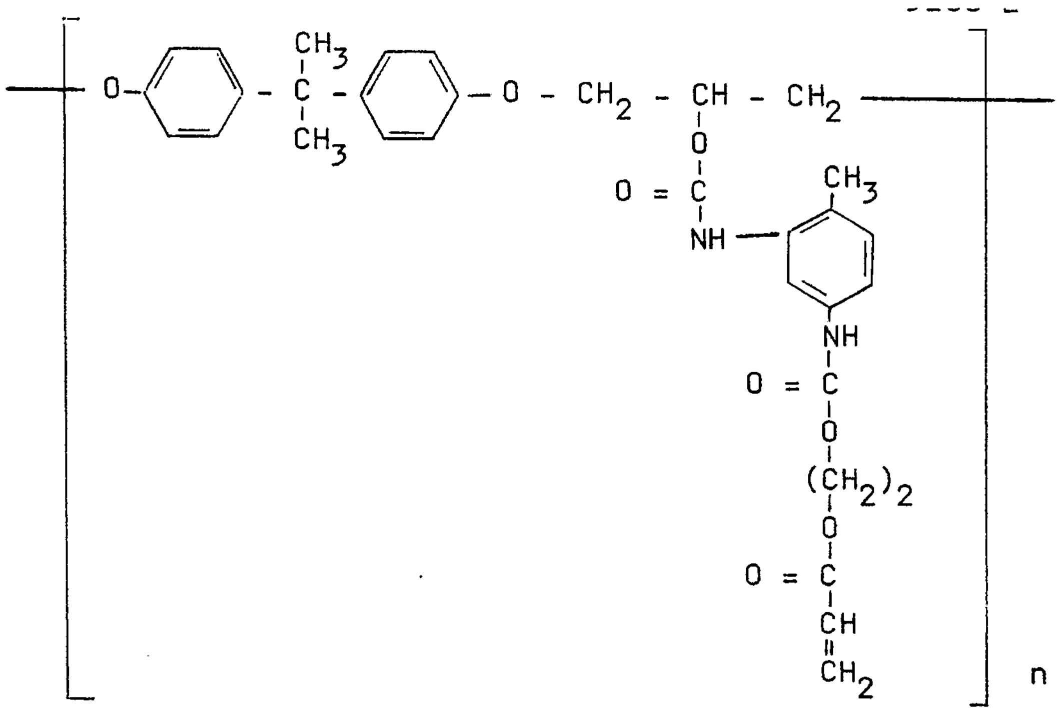

dabei ist n ≥ 50.

Die eingesetzten Phenoxyharze weisen vorzugsweise ein Molekulargewicht zwischen 15000 und 30000 auf.

Die Herstellung der erfindungsgemäßen strukturierten Schichten erfolgt, wie bereits ausgeführt, in der Weise, daß das Photopolymere in Form einer Schicht oder Folie auf ein Substrat aufgebracht und mit aktinischem Licht durch eine Maske belichtet oder durch Führen eines Licht-, Elektronen-, Laser- oder Ionenstrahls bestrahlt wird. Anschließend werden die nicht-belichteten bzw. nicht-bestrahlten Schicht- bzw. Folienteile herausgelöst oder abgezogen und die dabei erhaltenen strukturierten Schichten bzw. Reliefstrukturen gegebenenfalls getempert. Das Photopolymere wird dabei vorteilhaft in einem organischen Lösungsmittel gelöst auf das Substrat aufgebracht. Die Konzentration der Photolacklösung in gängigen Lösungsmitteln, wie Cyclohexanon, γ-Butyrolacton, N-Methylpyrrolidon und Gemischen davon, kann so eingestellt werden, daß mit bekannten Beschichtungsverfahren, wie Schleudern, Tauchen, Sprühen, Gießen, Rakeln, Bürsten oder Rollen, Schichtstärken von 0,01 bis ca. 500 µm erzeugt werden können. Zur Erzielung einer gleichmäßigen und guten Oberflächenqualität auf Substraten mit glatter Oberfläche haben sich das Gießverfahren (ein derartiges Verfahren ist beispielsweise aus der EP-PS 0 002 040 bekannt), das Rakeln und insbesondere die elektrostatische Sprühbeschichtung und das Schleuderbeschichten bei 300 bis 10000 Umdrehungen/Minute als vorteilhaft erwiesen. Bei unebenen Oberflächen, wie Leiterplatten mit Kupferleitbahnen auf der Oberfläche, sind Schleuderdrehzahlen von 300 bis 1500 vorteilhaft. Der Viskositätsbereich der für das Rakeln, das Sprühen und das Gießverfahren eingesetzten Lacklösungen liegt vorteilhaft zwischen 200 und 1500 mPa.s bei 23 °C.

Die auf das Substrat, das vorzugsweise aus Leiterplattenmaterial, Glas, Metall, Kunststoff oder Halbleitern besteht, aufgebrachte Photolackschicht kann bei Raumtemperatur, vorzugsweise bei Temperaturen von 50 bis 80 °C, in einem Stickstoff- oder Luftstrom vom Lösungsmittel befreit werden; dabei kann auch im Vakuum gearbeitet oder mit Infrarotstrahlern bzw. auf einer beheizten Platte getrocknet werden.

Zur Erzielung eines ausreichenden Löslichkeitsunterschiedes zwischen den bestrahlten und den nicht-bestrahlten Schicht-bzw. Folienteilen genügen beim erfindungsgemäßen Verfahren, bei Verwendung einer 350 W-Quecksilberhöchstdrucklampe, in Abhängigkeit von der Zusammensetzung und der Schichtstärke Belichtungszeiten zwischen 5 und 400 s. Nach dem Belichten werden, gegebenenfalls nach einem Nachtrocknungsprozeß, die nicht-belichteten Teile mit organischen Lösungsmitteln herausgelöst.

Die nach dem erfindungsgemäßen Verfahren hergestellten strukturierten Schichten bzw. Reliefstrukturen zeichnen sich durch Kantenschärfe, hohe Auflösung, eine rißfreie homogene Oberfläche und eine Wärmeformbeständigkeit aus, die auch den thermi schen und mechanischen Beanspruchungen eines Tauchlötprozesses standhält. Die Haftung zum Lot ist sehr gering, so daß, wie erwünscht, keine Lotperlen an der Polymerschicht hängenbleiben. Die erfindungsgemäß hergestellten strukturierten Schichten sind elastisch genug, um Cyceltests zwischen -65 und +125 °C ohne Rißbildung zu bestehen. Mit den strukturierten

Schichten abgedeckte Schaltungsoberflächen zeigen in Klimatests bei 40° C und 92 % Luftfeuchte unter Spannung (100 V) keine Leitbahnkorrosion. Derartige Schichten eignen sich also - neben der Anwendung als Lötstoppmasken - auch als wirksame und dauerhafte Schutzschichten gegen Feuchte- und Schadgaseinwirkung.

Die erfindungsgemäßen strukturierten Schichten eignen sich - wegen der herstellungsbedingt hohen Reinheit - auch zur Herstellung von Passivierungsschichten auf Halbleiterbauelementen, von Dünn- und Dickfilmschaltungen, von Lötschutzschichten auf Mehrlagenverschaltungen, von Isolierschichten als Bestandteil von Schichtschaltungen und von miniaturisierten Schutz- und Isolierschichten auf elektrisch leitenden und/oder halbleitenden und/oder isolierenden Basismaterialien, und allgemein zur Feinstrukturierung von Substraten und für Strukturübertragungsprozesse, wie Naß- und Trockenätzprozesse, stromlose oder galvanische Metallabscheidung und Aufdampfverfahren, sowie als Masken für die Ionenimplantation. Darüber hinaus eignen sich diese Schichten als Isolier- und Schutzschichten in der Elektrotechnik und in der Mikroelektronik, ferner als Dämpfungsmassen für Oberflächenwellenfilter, insbesondere Fernsehzwischenfrequenzfilter, sowie als ∝-Strahlenschutz auf den Zellenfeldern von Speicherbausteinen und als Orientierungsschichten in Flüssigkristalldisplays.

Anhand von Ausführungsbeispielen soll die Erfindung noch näher erläutert werden.

Beispiel 1

Zu 69 Masseteilen getrocknetes Dichlormethan werden unter Feuchteausschluß 40 Masseteile reines 2.4-Diisocyanatotoluol und zu der dabei erhaltenen Lösung dann bei Raumtemperatur unter Rühren langsam 29,2 Masseteile 2-Hydroxyethylacrylat getropft. Nach 24stündigem Stehen wird durch Titration der Isocyanatgruppen ein 99 %iger Umsatz ermittelt. Das photoreaktive Isocyanat wird mit 250 Masseteilen Petroleumbenzin aus der Reaktionslösung extrahiert und nach Entfernen des Extraktionsmittels als klare viskose Flüssigkeit isoliert; Ausbeute: 64,5 g, d.h. 93 % der Theorie.

Zu 35 Masseteilen des Phenoxyharzes Rütapox 0717 (Rütapox$^R$ ist ein Warenzeichen der Fa. Rütgerswerke AG) werden bei Raumtemperatur und unter Rühren 140 Masseteile trockenes γ-Butyrolacton, 137 Masseteile trockenes N-Methylpyrrolidon, 50 Masseteile des in der vorstehend beschriebenen Weise isolierten reinen photoreaktiven Monoisocyanats und 0,1 Masseteile Dibutylzinndilaurat gegeben. Nachdem das Gemisch 48 Stunden gerührt wurde, werden zur Reaktionslösung 7 Masseteile Ethanol gegeben. Nach weiteren 24 Stunden sind keine Isocyanatgruppen mehr nachweisbar.

Zu 100 Masseteilen des in der vorstehend beschriebenen Weise - als 23 %ige Harzlösung - hergestellten photoreaktiven Phenoxyharzes werden 0,65 Masseteile Benzoinisopropylether, 0,08 Masseteile Michler's Keton, 1,65 Masseteile Trimethylolpropantriacrylat und 0,3 Masseteile Vinyl-tris(β-methoxyethoxy)-silan gegeben. Die Lösung wird dann durch ein 5 µm-Filter druckfiltriert. Die Viskosität der dabei erhaltenen Lösung beträgt bei 23° C 830 mPa.s.

Durch Aufschleudern der Lösung bei 800 Umdrehungen/Minute auf eine mit einem Haftvermittler beschichtete Siliciumscheibe werden nach 30minütigem Trocknen bei 60° C in einem Umluftofen 23 µm dicke homogene Schichten erhalten, die nach einer Belichtung von 40 s mit einer 350 W-Quecksilberhöchstdrucklampe durch eine Maske mit γ-Butyrolacton/Xylol (Volumenverhältnis 1:2) und Nachspülen mit Xylol im Sprühverfahren zu konturenscharfen strukturierten Schichten entwickelt werden. Die Kantenausbildung und die Oberflächenqualität dieser Schichten werden durch einstündiges Tempern bei 150° C nicht beeinträchtigt. Die aufgelösten Strukturen liegen bei $< 20$ µm.

Eine mit bekannten käuflichen Flußmitteln behandelte photostrukturierte Schicht zeigt nach dem Lötbadtest bei 260° C und 20 s Tauchzeit eine homogene rißfreie Oberfläche. Das Lot perlt von der Lackoberfläche gut ab.

Beispiel 2

Zu einer Lösung von 110 Masseteilen Rütapox 0723 (50 %ige Lösung in Cyclohexanon/Ethylglycolacetat) in 114 Masseteilen γ-Butyrolacton werden 33 Masseteile reines Isocyanatoethylmethacrylat und 0,1 Masseteile Dibutylzinndilaurat gegeben, dann wird bei Raumtemperatur 30 Stunden lang gerührt; danach werden zur Reaktionslösung 9 Masseteile 2-Hydroxyethylmethacry-lat gegeben. Nach weiteren 24 Stunden werden 100 Masseteilen der Harzlösung 0,6 Masseteile Dichloracetophenon, 0,3 Masseteile Diethoxyacetophenon, 0,3 Masseteile Michler's Keton und 0,3 Masseteile Vinyl-tris(β-methoxyethoxy)-silan zugesetzt. Nachfolgend wird die Lösung durch ein 5 µm-Filter druckfiltriert.

Die filtrierte Lösung des photoreaktiven Phenoxyharzes wird bei 400 Umdrehungen/Minute auf eine Leiterplatten-Testplatte mit Kupferleitbahnen auf der Oberfläche geschleudert und danach 1/2 Stunde bei 70° C im Umluftofen getrocknet. Die Dicke der Lackschicht beträgt dann 50 µm. Nach Belichten mit einer 350 W-Quecksilberhöchstdrucklampe durch eine Maske für 10 s und nach 30minütigem Nachtrocknen bei 70° C im Umluftofen werden mit Cyclohexanon als Entwickler, wobei Wasser als Stopper verwendet wird, nach 35 s kantenscharfe strukturierte Schichten erhalten, die in ihrer Oberflächenqualität auch durch hundertmaliges Cyceln zwischen -65° und +125° C nicht beeinträchtigt werden. Diese Schichten widerstehen ebenso unbeschadet Schwall- und Tauchlötprozessen bei 260° C; das Lot perlt von der Oberfläche ab. Feuchtetests bei 40° C und 92 % Luftfeuchte und unter einer Spannung von 100 V zeigen keine Korrosion in den mit Lack abgedeckten Leitbahnbereichen.

Beispiel 3

Zu 113,2 Masseteilen getrocknetes Dichlormethan werden unter Feuchteausschluß 62 Masseteile 2.4-Diisocyanatotoluol und danach bei Raumtemperatur und unter Rühren langsam 51,2 Masseteile 2-Hydroxyethylmethacrylat getropft. Nach einer Reaktionsdauer von 24 Stunden bei Raumtemperatur wird der Isocyanatumsatz titrimetrisch zu 99 % ermittelt. Durch Zugabe von 450 Masseteilen Petroleumbenzin wird ein Niederschlag von weißen Kristallen erhalten. Die Ausbeute an reinem photoreaktiven Monoisocyanat beträgt 105 Masseteile bzw. 93 % der Theorie.

Zu einer Lösung von 95 Masseteilen des in der beschriebenen Weise hergestellten photoreaktiven Monoisocyanats in einem Gemisch von 140 Masseteilen γ-Butyrolacton, 105 Masseteilen N-Methylpyrrolidon und 0,01 Masseteilen Dibutylzinndilaurat werden unter Feuchteausschluß 125,2 Masseteile Phenoxyharzlösung (Rütapox 0723) gegeben und unter Rühren 12 Stunden lang bei 50° C zur Reaktion gebracht. Danach werden bei dieser Temperatur zur Reaktionslösung zunächst 8 Masseteile 2-Hydroxyethyl-methacrylat und nach weiteren 5 Stunden 4 Masseteile Ethanol gegeben. Nach 24 Stunden hat die Harzlösung eine Viskosität von 330 mPa.s bei 23° c.

Zu 100 Masseteilen der vorstehend beschriebenen Lösung werden 0,66 Masseteile Isopropylthioxanthon, 0,08 Masseteile Michler's Keton, 1,65 Masseteile Trimethylolpropantriacrylat und 0,3 Masseteile Vinyltriethoxysilan gegeben. Nach Filtration durch ein 5 µm-Filter bei einem Druck von 5 bar werden im Gießverfahren auf Substraten 20 µm dicke Lackschichten hergestellt und mit einer 350 W-Quecksilberhöchstdrucklampe 30 s durch eine Maske bestrahlt. Danach wird die belichtete Schicht bei 90° C 5 min. im Umluftofen nachgetrocknet. Die Strukturierung (Dauer: 10 s) erfolgt mittels eines Entwicklergemisches aus γ-Butyrolacton und Xylol (Volumenverhältnis 1:2), wobei mit Xylol abgestoppt wird. Die auf diese Weise hergestellte strukturierte Schicht ist lötbadresistent.

Beispiel 4

Zu einer Lösung von 66,8 Masseteilen reinem 2.4-Diisocyanatotoluol in 115,3 Masseteilen Dichlormethan wird bei Raumtemperatur unter Rühren und Feuchteausschluß ein Gemisch von 23,6 Masseteilen reinem 2-Hydroxyethylacrylat und 24,8 Masseteilen reinem 2-Hydroxyethylmethacrylat langsam zugetropft. Nach 40stündiger Reaktion bei Raumtemperatur wird durch Titration ein Isocyanatumsatz von 98 % ermittelt.

Die erhaltene Lösung des photoreaktiven Monoisocyanats wird mit einer Lösung von 107 Masseteilen Rütapox 0717 in 397 Masseteilen γ-Butyrolacton und mit 0,22 Masseteilen Dibutylzinndilaurat vereinigt. Nach 25stündiger Reaktion bei Raumtemperatur werden zur Reaktionslösung 15 Masseteile Ethanol gegeben. Nach weiteren 24 Stunden ist die Lösung für Beschichtungen gebrauchsfertig.

Zu 100 Masseteilen der in der geschilderten Weise hergestellten Lösung des Polyethers werden 1,2 Masseteile 2.4.6-Trimethylben-zoylphosphinoxid, 0,3 Masseteile Michler's Keton und 0,3 Masse-teile Vinyltriethoxysilan gegeben. Nach Filtration durch ein 5 µm-Filter bei einem Druck von 5 bar werden im Gießverfahren auf Kupfersubstraten 30 µm dicke Lackschichten hergestellt und mit einer 350 W-Quecksilberhöchstdrucklampe 30 s durch eine Maske bestrahlt. Nach Entwickeln mit dem Entwickler entsprechend Beispiel 2 für die Dauer von 35 s werden Strukturen mit glatter rißfreier Oberfläche erhalten, die unter Lötbadbedingungen auch bei 260° C stabil sind; das Lot haftet nicht an der Lackoberfläche, sondern perlt ab.

## Ansprüche

1. Verfahren zur Herstellung wärmebeständiger strukturierter Schichten durch Auftragen strahlungsempfindlicher löslicher Polymerer in Form einer Schicht oder Folie auf ein Substrat, Bestrahlen der Schicht bzw. Folie durch Negativvorlagen mit aktinischem Licht oder durch Führen eines Licht-, Elektronen-, Laser- oder Ionenstrahls, Entfernen der nicht-bestrahlten Schicht- bzw. Folienteile und gegebenenfalls durch nachfolgendes Tempern, unter Verwendung von Photopolymeren auf Polyetherbasis in Form von Additionsprodukten aus einem olefinisch ungesättigten Monoisocyanat und einem hydroxylgruppenhaltigen Phenoxyharz , **dadurch gekennzeichnet,** daß als olefinisch ungesättigtes Monoisocyanat ein methacrylatgruppenhaltiges Isocyanat oder ein Additionsprodukt von Hydroxyethyl(meth)acrylat an 2.4-Diisocyanatotoluol eingesetzt wird.

2. Verfahren nach Anspruch 1, **dadurch gekennzeichnet**, daß die Photopolymeren zusammen mit lichtoder strahlungsempfindlichen copolymerisationsfähigen Verbindungen, insbesondere acrylat- und methacrylatgruppenhaltigen Verbindungen, eingesetzt werden.

3. Verfahren nach Anspruch 1 oder 2, **dadurch gekennzeichnet** , daß die Photopolymeren zusammen mit Photoinitiatoren und/oder Photosensibilisatoren, insbesondere ∝-Halogenacetophenonen, Dialkoxyacetophenonen, Benzoylphosphinoxiden und Michler's Keton, eingesetzt werden.

4. Verfahren nach einem der Ansprüche 1 bis 3, **dadurch gekennzeichnet,** daß Phenoxyharze mit einem Molekulargewicht zwischen 15000 und 30000 verwendet werden.

5. Wärmebeständige strukturierte Schicht, hergestellt nach einem oder mehreren der Ansprüche 1 bis 4.

6. Verwendung der strukturierten Schicht nach Anspruch 5 als Lötstopp- und Isolierschicht mit dauerhafter Schutzfunktion in der Feinleitertechnik.

7. Verwendung der strukturierten Schicht nach Anspruch 5 als Resist mit intermediärer Schutzfunktion bei Strukturübertragungsprozessen durch Galvanisieren, Naß- und Trockenätzen sowie durch Ionenimplantation.

8. Verwendung der strukturierten Schicht nach Anspruch 5 als Schutz- und Isolierstoff in der Elektrotechnik, insbesondere in der Halbleitertechnik.

9. Verwendung der strukturierten Schicht nach Anspruch 5 als Dämpfungsmasse für Oberflächenwellenfilter.

10. Verwendung der strukturierten Schicht nach Anspruch 5 als ∝-Strahlenschutz auf den Zellenfeldern von Speicherbausteinen.

11. Verwendung der strukturierten Schicht nach Anspruch 5 als Orientierungsschicht in Flüssigkristalldisplays.

## Claims

1. Process for the production of heat-resistant, structured layers by means of the application of radiation-sensitive, soluble polymers in the form of a layer or foil on a substrate, the irradiation of the layer or foil through negative patterns with actinic light or by guiding a light, electron, laser or ion beam, the removal of the non-irradiated portions of the layer or foil, and possibly by means of subsequent tempering, with the use of polyether-based photopolymers in the form of addition products of an olefin-unsaturated monoisocyanate and a hydroxyl group-containing phenoxy resin, **characterised in that** a methacrylate group-containing isocyanate, or an addition product of hydroxyethyl(meth)acrylate and 2,4-diisocyanatotoluene is used as the olefin-unsaturated monoisocyanate.

2. Process according to claim 1, **characterised in that** the photopolymers are used together with light- or radiation-sensitive compounds capable of copolymerisation, in particular compounds containing

acrylate or methacrylate groups.

3. Process according to claim 1 or 2, **characterised in that** the photopolymers are used together with photo initiators and/or photo sensitizers, in particular alpha-halogen acetophenones, dialkoxy acetophenones, benzoylphosphine oxides and Michler's ketone.

4. Process according to one of claims 1 to 3, **characterised in that** phenoxy resins with a molecular weight between 15,000 and 30,000 are used.

5. Heat-resistant structured layer, produced according to one or several of claims 1 to 4.

6. Use of the structured layer according to claim 5 as a solder resist and insulation layer providing lasting protection in micro-conductor technology.

7. Use of the structured layer according to claim 5 as a resist providing intermediate protection in structure transfer processes carried out by galvanising, wet and dry etching, and ion implantation.

8. Use of the structured layer according to claim 5 as a protective and insulating material in electrical engineering, in particular in semiconductor technology.

9. Use of the structured layer according to claim 5 as a damping substance for surface wave filters.

10. Use of the structured layer according to claim 5 as alpha-ray protection on the cell fields of memory components.

11. Use of the structured layer according to claim 5 as an orientation layer in liquid crystal displays.

**Revendications**

1. Procédé de préparation de couches thermostables structurées, par dépôt de polymères solubles, sensibles au rayonnement, sous la forme d'une couche ou d'une feuille, sur un substrat, par exposition de la couche ou de la feuille à travers un modèle négatif à la lumière actinique ou par envoi d'un faisceau lumineux, d'électrons, laser ou d'ions, par suppression des parties de la couche ou de la feuille qui n'ont pas été exposées et, le cas échéant, par recuit ultérieur, en utilisant des photopolymères à base de polyéthers sous la forme de produits d'addition d'un monoisocyanate à insaturation oléfinique et d'une rêsine phénoxylique contenant des groupes hydroxyle, caractérisé en ce qu'il consiste à utiliser, comme monoisocyanate à insaturation oléfinique, un isocyanate contenant un groupes méthacrylate ou un produit d'addition de (méth)acrylate d' hydroxyéthyle et de 2,4-diisocyanatoluène.

2. Procédé suivant la revendication 1, caractérisé en ce qu'il consiste à utiliser les photopolymères avec des composés qui sont susceptibles d'être copolymérisés et qui sont sensibles à la lumière ou au rayonnement, notamment des composés contenant des groupes acrylate ou méthacrylate.

3. Procédé suivant la revendication 1 ou 2, caractérisé en ce qu'il consiste à utiliser les photopolymères avec des photoinitiateurs et/ou des photosensibilisateurs, notamment des a-haloacétophénones, des dialcoxy acétophénones, des oxydes de benzoylphosphine et la cétone de Michler.

4. Procédé suivant l'une des revendications 1 à 3, caractérisé en ce qu'il consiste à utiliser des résines phénoxyliques d'une masse moléculaire comprise entre 15000 et 30000.

5. Couche thermostable structurée, préparée suivant l'une ou plusieurs des revendications 1 à 4.

6. Utilisation de la couche structurée suivant la revendication 5, comme couche d'arrêt de soudure et d'isolation à fonction de protection durable dans la technique des conducteurs minces.

7. Utilisation de la couche structurée suivant la revendication 5, comme résist à fonction intermédiaire de

protection, dans des processus de transfert de structure par galvanisation, par gravure en voie humide et en voie sèche, ainsi que par implantation d'ions.

**8.** Utilisation de la couche structurée suivant la revendication 5, comme substance de protection et d'isolation en électrotechnique, notamment dans la technique des semiconducteurs.

**9.** Utilisation de la couche structurée suivant la revendication 5, comme masse d'amortissement pour un filtre à ondes de surface.

**10.** Utilisation de la couche structurée suivant la revendication 5, comme protection vis-à-vis du rayonnement a sur les champs de cellules de modules de mémoire.

**11.** Utilisation de la couche structurée suivant la revendication 5, comme couche d'orientation dans des dispositifs d'affichage à cristaux liquides.